# EUROPEAN PATENT SPECIFICATION

(11) **EP 3 964 252 B1**
(45) Date of publication and mention of the grant of the patent: **07.10.2026**
(21) Application number: 21197374.8
(22) Date of filing: 30.05.2018
(51) Int. Cl.: A61M 5/32, A61M 5/142

(54) **NEEDLE SHIELD FOR INJECTION NEEDLE RETRACTION**
NADELSCHUTZ FÜR INJEKTIONSNADELEINZUG
PROTECTION D'AIGUILLE POUR RÉTRACTION D'AIGUILLE D'INJECTION

(30) Priority: 30.05.2017 US 201762512474 P
(43) Date of publication of application: 09.03.2022
(62) Document number(s) of the earlier application(s) in accordance with Art. 76 EPC: 18730955.4 / 3 630 237
(73) Proprietor: West Pharma. Services IL, Ltd., 4366411 Ra'anana (IL)
(72) Inventor: YIGAL, Gil, 70800 Gan Yavne (IL); BAR-EL, Yossi, 71947 Beit Arye (IL)
(74) Representative: Petraz, Gilberto Luigi

(56) References cited:
- WO-A1-2017/210448
- WO-A2-02/02165
- US-A1- 2013 253 434

## Description

### BACKGROUND OF THE DISCLOSURE

The present disclosure is generally directed to a needle shield, and, more particularly, to a needle shield employed in an injector for covering and retracting an injection needle post injection.

An injector, such as, for example, a drug injector, is typically engaged with a user's skin to perform an injection. Thereafter, the injector is withdrawn from the user's skin, leaving the injection needle thereof exposed. It is well documented that exposure of a used needle is dangerous as an individual (whether the user or another individual) may be pricked by the exposed, used needle, causing injury or harm, whether by the physical prick, by exposing an individual to a drug that may cause a reaction, or by blood transmitted disease.

In certain prior wearable injector devices, the injection needle is non-retractable, but rather a needle shield is employed to cover the injection needle after injection. The needle shield may be maintained propped open by the injection needle. Subsequent collapse of the needle shield bends the injection needle into a secure area within the needle shield. In rare cases, however, it has been found that bending of the needle may nevertheless potentially result in a stick hazard. US2013/253434 discloses a device for preventing a needle stick hazard in the event of a collapse of a protective needle flap of a portable drug pump. The device includes a needle guide, a secure space and/or a shield. A point of a needle is deflected into a secure shielded space upon collapse of the protective flap. Also provided is an exposing position wherein the needle protrudes through an opening in the flap. WO2017/210448 represents state of the art according to Article 54(3) EPC. It discloses a system for safeguarding a needle point, that may be moved between a protected retracted position, an active extended position and/or an intermediate position. In the retracted position, the needle point is protected inside a housing, and in the extended position it is exposed. In the intermediate position, the needle point is partially protected and/or concealed by a shield and/or an indentation.

Therefore, it would be advantageous to manufacture an injector having a needle shield, together configured such that collapse of the needle shield predominantly retracts the injection needle back toward the retracted (pre-injection) position thereof.

### BRIEF SUMMARY OF THE DISCLOSURE

Briefly stated, one aspect of the present disclosure is directed to an injector according to annexed independent claim 1.

Other advantageous features are defined in the dependent claims.

### BRIEF DESCRIPTION OF THE DRAWINGS

The following detailed description of aspects of the disclosure will be better understood when read in conjunction with the appended drawings. It should be understood, however, that the disclosure is not limited to the precise arrangements and instrumentalities shown. In the drawings:
Fig. 1 is an elevational view of a wearable injector, having a needle shield in accordance with a first embodiment of the present disclosure movably mounted thereto;
Fig. 2 is a cross-sectional view of the injector and needle shield of Fig. 1, taken along the sectional line 2-2 of Fig. 1, with an injection needle of the injector in a retracted position thereof and the needle shield in a second position thereof;
Fig. 3 is a top and side perspective view of the needle shield of Fig. 1;
Fig. 4 is an enlarged, partial cross-sectional view of the injector and needle shield of Fig. 1, taken along the sectional line 2-2 of Fig. 1, with the injection needle in a injection position thereof and the needle shield in a first position thereof;
Fig. 5 is an enlarged, partial cross-sectional view of the injector and needle shield of Fig. 1, taken along the sectional line 2-2 of Fig. 1, with the injection needle in the injection position thereof and the needle shield returning from the first position thereof to the second position thereof;
Fig. 6 is an enlarged, partial cross-sectional view of the injector and needle shield of Fig. 1, taken along the sectional line 2-2 of Fig. 1, with the injection needle in the injection position thereof and the needle shield further traveled from the first position thereof to the second position thereof relative to Fig. 5;
Fig. 7 is an enlarged, partial cross-sectional view of the injector and needle shield of Fig. 1, taken along the sectional line 2-2 of Fig. 1, with the injection needle in the injection position thereof and the needle shield in the second position thereof;
Fig. 8 is an enlarged, partial cross-sectional view of the injector and needle shield of Fig. 1, taken along the sectional line 2-2 of Fig. 1, with needle shield moved from the second position thereof substantially back to the first position thereof and axially translating the injection needle therewith substantially back to the retracted position thereof;
Fig. 9 is an enlarged, partial cross-sectional view of the an injector and a needle shield in accordance with a second embodiment of the present disclosure, taken along the sectional line 2-2 of Fig. 1, with the needle hub being retracted by the needle shield (in broken line) and the needle hub clearing the needle shield (in solid line); and
Fig. 10 is an enlarged, partial cross-sectional view of the injector and the needle shield of Fig. 9, taken along the sectional line 2-2 of Fig. 1, with needle shield being moved from the second position thereof back to the first position thereof and axially translating the needle hub therewith back to the retracted position thereof.

### DETAILED DESCRIPTION OF THE DISCLOSURE

Certain terminology is used in the following description for convenience only and is not limiting. The words "lower," "bottom," "upper" and "top" designate directions in the drawings to which reference is made. The words "inwardly," "outwardly," "upwardly" and "downwardly" refer to directions toward and away from, respectively, the geometric center of the injector, and designated parts thereof, in accordance with the present disclosure. Unless specifically set forth herein, the terms " a, " "an" and "the" are not limited to one element, but instead should be read as meaning "at least one." The terminology includes the words noted above, derivatives thereof and words of similar import.

It should also be understood that the terms "about," "approximately," "generally," "substantially" and like terms, used herein when referring to a dimension or characteristic of a component of the disclosure, indicate that the described dimension/characteristic is not a strict boundary or parameter and does not exclude minor variations therefrom that are functionally similar. At a minimum, such references that include a numerical parameter would include variations that, using mathematical and industrial principles accepted in the art (e.g., rounding, measurement or other systematic errors, manufacturing tolerances, etc.), would not vary the least significant digit.

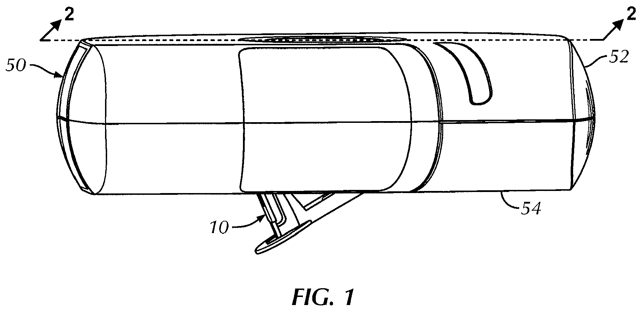

Referring to the drawings in detail, wherein like numerals indicate like elements throughout, there is shown in Figs. 1-8 an injection needle shield, generally designated 10, in accordance with a first embodiment of the present disclosure. Generally, the injection needle shield 10 is employed with a wearable injector (patch injector) 50, such as, for example, without limitation, a wearable drug injector, but the disclosure is not so limited. At a minimum, the injection needle shield 10 may alternatively be employed in other injector configurations.

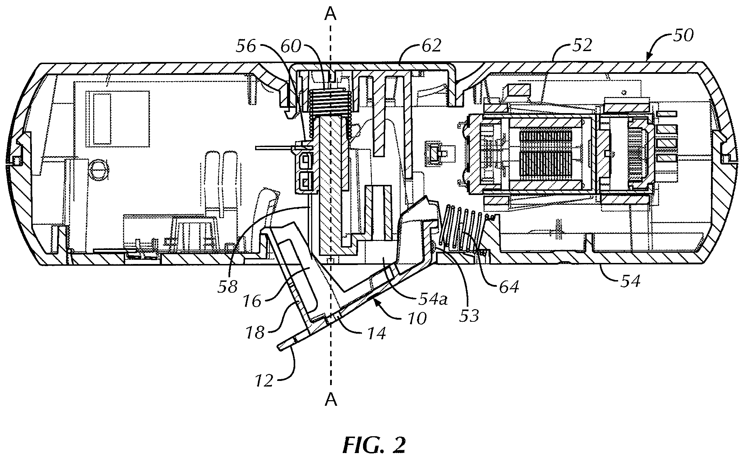

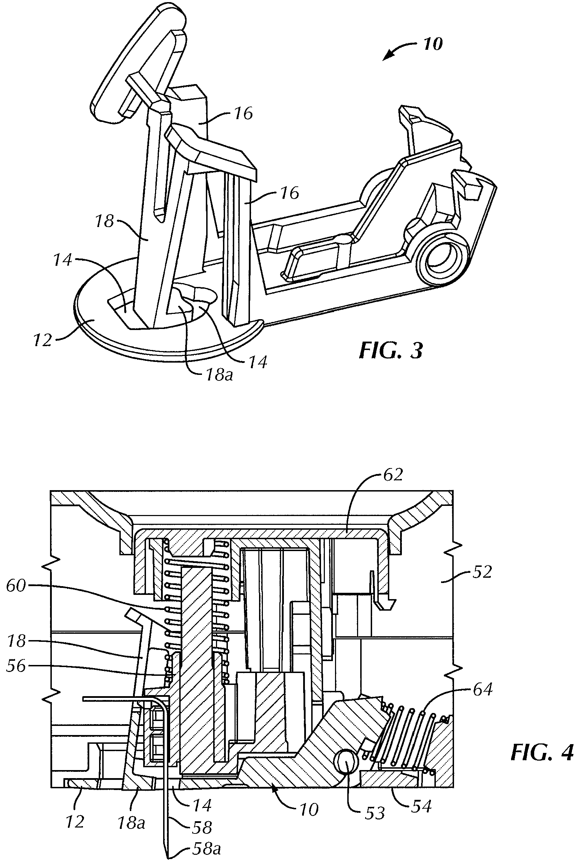

As should be understood by those of ordinary skill in the art, and as best shown in Fig. 2, an injector 50 generally comprises a housing 52 having a surface 54 configured to contact a skin surface of a user (not shown), e.g., a patient, the surface 54 having an opening 54a therein. As shown, a needle hub 56, e.g., a polymeric needle hub 56, is movably mounted within the injector housing 52 and an injection needle 58 is supported by the movable needle hub 56. In one non-limiting example, the injection needle 58 may be at least a 27 gauge needle. The needle hub 56 and the injection needle 58 are axially translatable along an axis A, extending substantially perpendicularly to the surface 54, between a retracted position (Fig. 2), wherein at least a tip 58a of the injection needle 58 is contained within the injector housing 52, and an injection position (Figs. 4-6), wherein at least the tip 58a of the injection needle 58 protrudes from the injector housing 52 through the opening 54a and through an aperture 14 of the needle shield 10 (as will be explained in further detail below).

A biasing member 60 is operatively connected with the injection needle 58 to drive the needle hub 56 and the injection needle 58 from the retracted position to the injection position. In the illustrated embodiment, the biasing member 60 takes the form of a coil spring expandable from a contracted state (Fig. 2) to an expanded state (Fig. 4). In the illustrated embodiment, the coil spring 60 is mounted between the needle hub 56 and a depressible activation button assembly 62 of the injector 50, i.e., the spring 60 abuts the activation button assembly 62 at one end and abuts the needle hub 56 at an opposing end. In the contracted, i.e., energy storing, state thereof, the coil spring 60 is prevented from driving the needle hub 56 and the injection needle 58 into the injection position. Upon release into the expanded, i.e., an energy releasing, state thereof (generally by depressing the activation button assembly 62), the coil spring 60 drives the needle hub 56 and the injection needle 58 into the injection position. As should be understood by those of ordinary skill in the art, the biasing member 60 may alternatively take the form of other members capable of storing and releasing a biasing force. Non-limiting examples include other springs (e.g., torsion or leaf springs), elastic bands, and the like. Alternatively, the biasing member 60 may take the form of an actuator configured to apply a translational force onto the needle hub 56 and the injection needle 58.

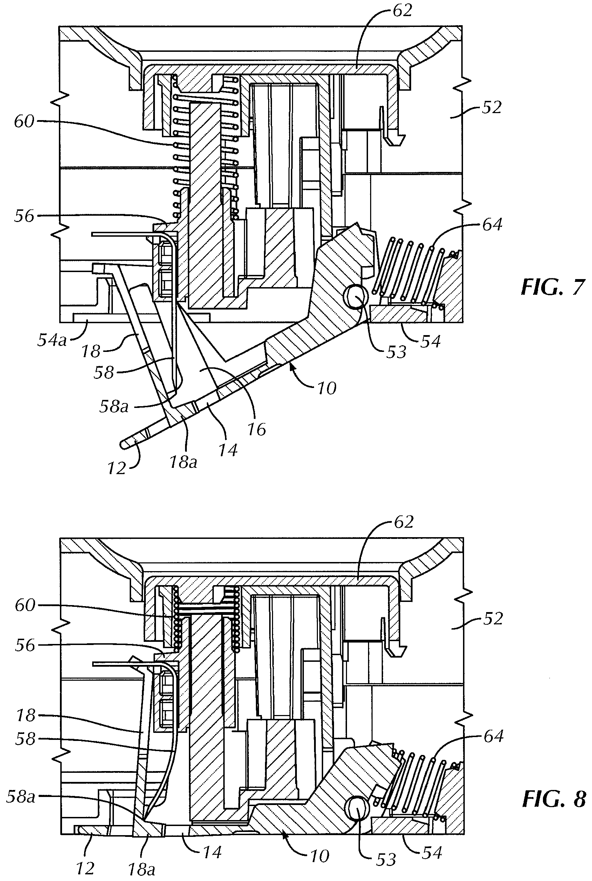

Turning to the needle shield 10, the needle shield 10, e.g., a polymeric needle shield 10, is movably connected to the injector housing 52 in a manner well understood by those of ordinary skill in the art. In the illustrated embodiment, the needle shield 10 is pivotably attached to the injector housing 52, e.g., via a pin connector 53 proximate an end of the needle shield 10, proximate the opening 54a of the skin contacting surface 54. The needle shield 10 is movable between a first position and a second position. In the first position (Fig. 4), the needle shield 10 extends generally flush with the skin contacting surface 54 of the injector 50, but the disclosure is not so limited. In the second position (Figs. 1, 2, 7), the needle shield 10 is pivoted away, i.e., downwardly, with respect to the axis A, from the skin contacting surface 54. In the second position, the aperture 14 of the needle shield 10 is elevationally lower than the tip 58a of the injection needle 58, with respect to the axis A, in any position of the injection needle 58. As will be explained in further detail below, when the needle shield 10 is in the second position and the injection needle 58 is in the injection position, the needle shield 10 covers the tip 58a of the injection needle 58 (Fig. 7). In the illustrated embodiment, the needle shield 10 is biased by a coil spring 64 to the second position thereof, but the disclosure is not so limited. Alternatively, the needle shield 10 may be biased into the second position thereof by another form of biasing member (such as, for example, without limitation, as identified above as alternative biasing members 60) or merely by gravitational force.

As shown best in Fig. 3, the needle shield 10 includes a base surface 12 defining the aperture 14 therein. The needle shield 10 further includes at least one post 16 (a pair of posts 16 in the illustrated embodiment) extending upwardly from the base surface 12. A cantilevered arm 18 extends downwardly from the post(s) 16 toward the base surface 12. The cantilevered arm 18 includes a flanged free end 18a, i.e., the free end of the arm 18 comprises a flange 18a extending generally laterally relative to the arm 18. The cantilevered arm 18 is dimensioned such that the flanged end 18a is positioned in the needle shield aperture 14 and the laterally extending flange 18a is angled to extend substantially along the same plane as the plane of extension of the base surface 12, thereby partially blocking the needle shield aperture 14.

Prior to placement of the injector 50 on the skin surface of a user, the injection needle 58 is blocked from extending through the needle shield aperture 14 (Fig. 2). That is, when the needle shield 10 is in the second position, the injection needle 58 is either not axially aligned with the aperture 14 or the flanged free end 18a of the cantilevered arm 18 blocks the pathway of the injection needle 58 through the aperture 14. Conversely, when the injector 50 is placed on the skin surface of a user (prior to injection), moving the needle shield 10 to the first position thereof (against the force biasing the needle shield 10 into the second position), the needle shield aperture 14 axially aligns with the injection needle 58. That is, when the needle shield 10 is moved to the first position thereof and the injection needle 58 is in the retracted position thereof, a portion of the needle shield aperture 14 unblocked by the flanged free end 18a of the cantilevered arm 18 is axially aligned with the injection needle 58. The flanged end 18a clears the injection needle 58 in such configuration. Accordingly, when the injector 50 is placed on the user's skin surface, the injection needle 58 may be moved to the injection position (Fig. 4), such that the injection needle 58 extends through the opening 54a of the injector 50, through the needle shield aperture 14 and into the user as the injection needle 58 is driven into the injection position thereof.

After injection, e.g., of medicament, is complete, the user (or another individual) removes the injector 50 from the skin surface of the user. Accordingly, as the injector 50 is removed from the skin surface (and while the injection needle 58 remains in the injection position thereof), the biasing force (either gravitational force or the biasing force of the spring 64) also returns the needle shield 10 to the second position thereof. Progressive movement of the needle shield 10 under the biasing force from the first position (where the injection needle 58 is aligned with an unblocked portion of the needle shield aperture 14) to the second position (wherein the injection needle 58 is either not axially aligned with the needle shield aperture 14 or the flanged free end 18a of the cantilevered arm 18 blocks the pathway of the injection needle 58 through the aperture 14) progressively moves at least the unblocked portion of the needle shield aperture 14 out of axial alignment with the injection needle 58.

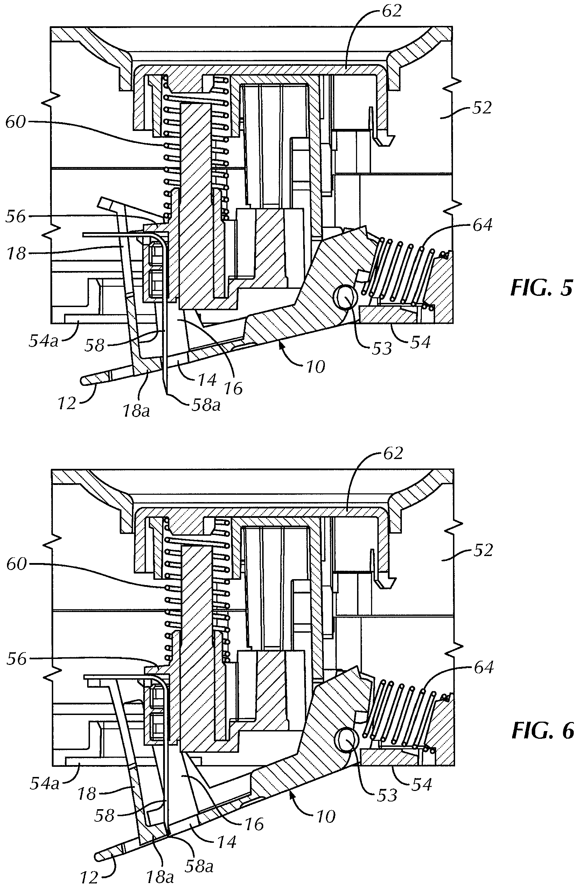

As shown between Figs. 5 and 6, the flanged end 18a of the cantilevered arm 18 comes into contact with the injection needle 58 during needle shield 10 movement from the first position (Fig. 4) toward the second position (Fig. 7) with the injection needle 58 in the injection position. The cantilevered arm 18 is constructed to be elastically flexible, and is more flexible than the injection needle 58. That is, the injection needle 58 is constructed to define a greater bending stiffness, i.e., resistance against bending deformation, than the cantilevered arm 18, in a manner well understood by those of ordinary skill in the art. For example, the cantilevered arm 18 may be constructed of a polymeric material and the injection needle 58 may be constructed of a metal material that exhibits a greater bending stiffness than the bending stiffness of the polymeric material of the cantilevered arm 18. The cantilevered arm 18 is, therefore, more deflectable than the injection needle 58.

Accordingly, when the injection needle 58 is in the injection position and comes into contact with the flanged end 18a of the cantilevered arm 18 (Fig. 5), as the needle shield 10 moves from the first position back to the second position, the injection needle 58 deflects (Fig. 6), i.e., elastically flexes, the cantilevered arm 18 out of an unflexed resting position thereof. Deflection of the cantilevered arm 18 temporarily moves the flanged end 18a aside, enlarging the unblocked portion of the needle shield aperture 14, to permit the needle shield 10 to pivot beyond the injection needle 58 and reach the second position thereof. As the needle shield 10 clears the tip 58a of the injection needle 58 and the injection needle 58 disengages the cantilevered arm 18, the cantilevered arm 18 returns to the unflexed resting position thereof (Fig. 7). As shown in Fig. 7, return of the cantilevered arm 18 to the unflexed resting position thereof when the needle shield 10 returns to the second position thereof results in the flanged free end 18a of the cantilevered arm 18 returning to blocking the pathway of the injection needle 58 through the needle shield aperture 14.

As explained previously, the needle shield 10 covers the tip 58a of the injection needle 58 when the needle shield 10 is in the second position and the injection needle 58 is in the injection position, thereby protecting the user or other individual from inadvertent contact with the tip 58a of the used injection needle 58 that may otherwise injure or infect the individual. After the injector 50 is removed from the skin surface of the user, and the needle shield 10 returns to the second position thereof to cover the injection needle tip 58a, however, inadvertent subsequent contact with the needle shield 10 with a force countering and overcoming the biasing force of the spring 64 moves the needle shield 10 back toward the first position thereof, i.e., collapse the needle shield.

As shown in Fig. 8, subsequent collapse of the needle shield 10 from the second position toward the first position thereof, advantageously axially translates the needle hub 56 and the injection needle 58 back toward the retracted position thereof to preclude the injection needle 58 from potentially extending beyond the needle shield 10. As shown in Fig. 8, the flanged free end 18a of the cantilevered arm 18 directly contacts the injection needle 58, e.g., contacts the tip 58a, upon such movement. In some embodiments, the injection needle 58 defines a greater stiffness than the stiffness of the coil spring 60 in a manner well understood by those of ordinary skill in the art. For example, the injection needle 58 may be constructed of a material that defines a stiffness greater than a stiffness of the coil spring 60 and/or a coil spring 60 may be selected that defines a weaker stiffness than the injection needle 58.

Accordingly, as shown in Fig. 8, contact of the needle shield 10 with the injection needle 58 during subsequent collapse of the needle shield 10 from the second position to the first position pushes the injection needle 58 and the needle hub 56 back toward the retracted position thereof, compressing the coil spring 60 back toward the contracted state, rather than merely bending the injection needle 58. In some cases, the injection needle 58 may also bend at least slightly, while moving back toward the injector housing 52 in addition to predominantly contracting the coil spring 60 to retract the needle hub 56 and the injection needle 58. Alternatively, the injection needle 58 may be supported against bending to cause retraction of the needle hub 56 and the injection needle 58 rather than bending of the injection needle 58. For example, the cantilevered arm 18 may be positioned to reduce or prevent bending of the injection needle 58. As should be understood by those of ordinary skill in the art, however, the needle shield 10 may be configured such that a different portion of the shield 10, e.g., the base surface 12, contacts the injection needle 58 upon such movement to axially translate the needle hub 56 and the injection needle 58 back toward the retracted position.

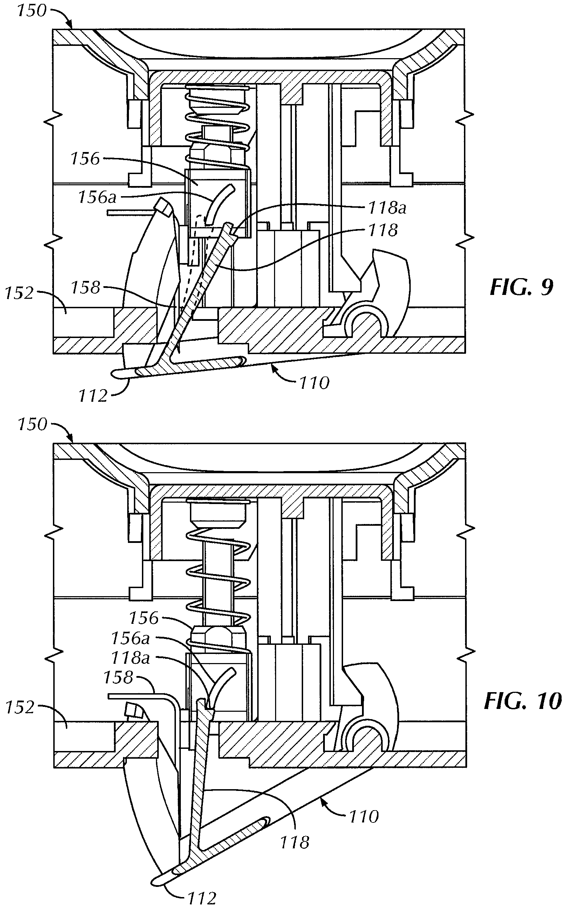

Figs. 9-10 illustrate a second embodiment of the needle shield 110. The reference numerals of the second embodiment (for both the injector 150 and the needle shield 110) are distinguishable from those of the above-described first embodiment (Figs. 1-8) by a factor of one-hundred (100), but otherwise indicate the same elements as indicated above, except as otherwise specified. The needle shield 110 of the present embodiment is substantially similar to that of the earlier embodiment. Therefore, the description of certain similarities and modes of operation between the embodiments may be omitted herein for the sake of brevity and convenience, and, therefore, is not limiting.

One difference of the needle shield 110 over the embodiment of Figs. 1-8, is that when the needle shield 110 is in the second position thereof and the needle hub 156 and the injection needle 158 are in the injection position, movement of the needle shield 110 back toward the first position indirectly axially translates (retracts) the injection needle 158 back toward the retracted position.

As shown in Figs. 9 and 10, the needle shield 110 comprises a cantilevered arm 118 extending upwardly from the base surface 112 of the needle shield 110 toward the needle hub 156. The cantilevered arm 118 defines a flanged free end 118a at an upper end thereof. That is, a flange 118a extends laterally from the cantilevered arm 18 at, or proximate, the upper free end thereof. The cantilevered arm 118 is configured to engage the needle hub 156, rather than the injection needle 158, during movement of the needle shield 110 from the second position thereof toward the first position thereof when the injection needle 158 is in the injection position, thereby axially translating the needle hub 156 back into the injector housing 152 to retract the needle hub 156 and the injection needle 158.

Conversely, prior to injection (Fig. 9 - arm 118 drawn in solid line), the needle hub 156 clears the cantilevered arm 118. That is, the cantilevered arm 118 is positioned such that when the needle hub 156 and the injection needle 158 are in the retracted position, the cantilevered arm 118 does not engage, or otherwise interfere with, the needle hub 156 during movement of the needle shield 110 from the second position thereof to the first position thereof, e.g., while initially placing the injector 150 on the body of the user. Thereafter the cantilevered arm 118 also does not interfere with movement of the needle hub 156 and the injection needle 158 from the retraction position to the injection position for injection.

After injection, when the injector 150 is removed from the user's skin and the needle shield 110 has returned to the second position thereof, if the needle shield 110 is pushed back toward the first position, as shown in Fig. 10, the cantilevered arm 118 contacts the needle hub 156 and retracts the needle hub 156 and the injection needle 158 toward the retracted position (Fig. 9 - arm 118 drawn in broken lines). In the illustrated embodiment, the needle hub 156 comprises a flanged member 156a extending therefrom configured to engage the flanged end 118a of the cantilevered arm 118, but the disclosure is not so limited.

It will be appreciated by those skilled in the art that changes could be made to the embodiments described above without departing from the broad inventive concept thereof. It is understood, therefore, that this disclosure is not limited to the particular embodiments disclosed, but it is intended to cover modifications within the scope of the appended claims.

## Claims

1. An injector (50, 150) comprising:
an injector housing (52, 152) having a surface (54, 154) configured to contact a skin surface of a user, the surface (54, 154) having an opening (54a, 154a) therein;
a needle hub (56, 156) movably mounted within the injector housing (52, 152);
an injection needle (58, 158) supported by the movable needle hub (56, 156), the needle hub (56, 156) and the injection needle (58, 158) being axially translatable between a retracted position, wherein at least a tip (58a, 158a) of the injection needle (58, 158) is contained within the injector housing (52, 152), and an injection position, wherein at least the tip (58a, 158a) of the injection needle (58, 158) protrudes from the injector housing (52, 152);
a needle shield (10, 110) comprising a surface (12), the surface (12) having a needle shield aperture (14, 114) and a cantilevered arm (18) extending therefrom, the needle shield (10, 110) being pivotably connected to the injector housing (52, 152) proximate to the opening (54, 154a) of the surface (54, 154) and movable between a first position and a second position; and
a biasing member (60) operatively connected with the injection needle (58, 158) to axially translate the needle hub (56, 156) and the injection needle (58, 158) from the retracted position to the injection position,
**characterized in that**:
the needle hub (56, 156) and the injection needle (58, 158) axially translate along an axis from the retracted position to the injection position;
the cantilevered arm (18) has a flanged free end (18a) partially blocking the needle shield aperture (14, 114), the injection needle (58, 158) extends through the needle shield aperture (14, 114) in the injection position of the injection needle (58, 158) and the first position of the needle shield (10, 110), the flanged free end (18a) clearing the injection needle (58, 158) in the first position of the needle shield (10, 110), the flanged free end (18a) blocking the injection needle (58, 158) from extending through the needle shield aperture (14, 114) in the second position of the needle shield (10, 110), and the injection needle (58, 158) elastically deflecting the cantilevered arm (18) to move the flanged free end (18a) aside and enlarge the needle shield aperture (14, 114) to permit the needle shield (10, 110) to pivot beyond the injection needle (58, 158) and reach the second position,
when the needle shield (10, 110) is in the second position and the needle hub (56, 156) and the injection needle (58, 158) are in the injection position, the flanged free end (18a) of the needle shield (10, 110) covers the tip (58a, 158a) of the injection needle (58, 158) and subsequent movement of the needle shield (10, 110) toward the first position axially translates the needle hub (56, 156) and the injection needle (58, 158) back toward the retracted position from the injection position, along the axis, and
the injection needle (58, 158) defines a stiffness greater than a stiffness of the biasing member.

2. The injector (50, 150) of claim 1, wherein the biasing member (60) applies a biasing force to drive the needle hub (56, 156) and the injection needle (58, 158) from the retracted position to the injection position, and when the needle shield (10, 110) is in the second position and the needle hub (56, 156) and the injection needle (58, 158) are in the injection position, subsequent movement of the needle shield (10, 110) from the second position toward the first position, with a force countering and overcoming the biasing force of the biasing member, axially translates the needle hub (56, 156) and the injection needle (58, 158) back toward the retracted position.

3. The injector (50, 150) of claim 1 or claim 2, wherein when the needle shield (10, 110) is in the second position and the needle hub (56, 156) and the injection needle (58, 158) are in the injection position, the needle shield (10, 110) directly contacts the injection needle to axially translate the needle hub (56, 156) and the injection needle back toward the retracted position, upon subsequent movement of the needle shield (10, 110) from the second position toward the first position.

4. The injector (50, 150) of at least one of the preceding claims, wherein when the needle shield (10, 110) is in the second position and the needle hub (56, 156) and the injection needle (58, 158) are in the injection position, the flanged free end of the cantilevered arm contacts the tip (58, 158) of the injection needle (58, 158) to axially translate the needle hub (56, 156) and the injection needle (58, 158) back toward the retracted position, upon subsequent movement of the needle shield toward the first position.

5. The injector (50, 150) of at least one of the preceding claims, wherein the cantilevered arm is elastically flexible, and wherein the cantilevered arm is more flexible than the injection needle (58, 158).

6. The injector (50, 150) of at least one of the preceding claims, wherein when the injection needle (58, 158) is in the injection position, the injection needle (58, 158) engages and elastically flexes the cantilevered arm out of an unflexed resting position thereof during movement of the needle shield (10, 110) from the first position to the second position, and wherein the injection needle (58, 158) disengages the cantilevered arm in the second position of the needle shield (10, 110), whereby the cantilevered arm returns to the unflexed resting position thereof.

7. The injector (50, 150) of at least one of the preceding claims, wherein when the injection needle (58, 158) is in the injection position, the injection needle (58, 158) engages the flanged free end of the cantilevered arm during movement of the needle shield (10, 110) from the first position to the second position.

8. The injector (50, 150) of at least one of the preceding claims, wherein when the needle shield (10, 110) is in the second position and the needle hub (56, 156) and the injection needle (58, 158) are in the injection position, subsequent movement of the needle shield (10, 110) toward the first position indirectly axially translates the injection needle (58, 158) back toward the retracted position.

9. The injector (50, 150) of claim 8, wherein when the needle shield (10, 110) is in the second position and the needle hub (56, 156) and the injection needle (58, 158) are in the injection position, subsequent movement of the needle shield (10, 110) toward the first position engages the arm of the needle shield (10, 110) with the needle hub (56, 156) to axially translate the needle hub (56, 156) and the injection needle (58, 158) back toward the retracted position.

10. The injector (50, 150) of claim 9, wherein the needle hub (56, 156) comprises a flanged member, and wherein when the needle shield (10, 110) is in the second position and the needle hub (56, 156) and the injection needle (58, 158) are in the injection position, subsequent movement of the needle shield (10, 110) toward the first position engages cantilevered arm with the flanged member of the needle hub (56, 156) to axially translate the needle hub (56, 156) and the injection needle (58, 158) back toward the retracted position.

11. The injector (50, 150) of claim 9, wherein the flanged free end (18a) is configured to engage with the needle hub (56, 156).

12. The injector (50, 150) of at least one of the preceding claims, wherein the needle shield (10, 110) is generally flush with surface of the injector housing (52, 152) in the first position, and the needle shield (10, 110) is pivoted away from the surface of the injector housing (52, 152) in the second position.

13. The injector (50, 150) of at least one of the preceding claims, wherein the needle shield (10, 110) is biased by a force into the second position thereof.

14. The injector (50, 150) of at least one of the preceding claims, wherein the biasing member (60) is a coil spring expandable from a contracted state to an expanded state to axially translate the needle hub (56, 156) and the injection needle (58, 158) from the retracted position to the injection position.

15. The injector (50, 150) of claim 14, wherein when the needle shield (10, 110) is in the second position and the needle hub (56, 156) and the injection needle (58, 158) are in the injection position, subsequent movement of the needle shield (10, 110) toward the first position that axially translates the needle hub (56, 156) and the injection needle (58, 158) back toward the retracted position also compresses the coil spring back toward the contracted state.

## Patentansprüche

1. Injektor (50, 150) umfassend:
ein Injektorgehäuse (52, 152), das eine Oberfläche (54, 154) hat, die dafür ausgelegt ist, eine Hautoberfläche eines Benutzers zu berühren, wobei die Oberfläche (54, 154) eine Öffnung (54a, 154a) darin aufweist;
einen Nadelansatz (56, 156), der innerhalb des Injektorgehäuses (52, 152) beweglich montiert ist;
eine Injektionsnadel (58, 158), die vom beweglichen Nadelansatz (56, 156) abgestützt ist, wobei der Nadelansatz (56, 156) und die Injektionsnadel (58, 158) zwischen einer eingezogenen Stellung, in der zumindest eine Spitze (58a, 158a) der Injektionsnadel (58, 158) innerhalb des Injektorgehäuses (52, 152) enthalten ist, und einer Einspritzstellung, in der zumindest die Spitze (58a, 158a) der Injektionsnadel (58, 158) aus dem Injektorgehäuse (52, 152) herausragt, axial verschiebbar sind;
einen Nadelschutz (10, 110) umfassend eine Oberfläche (12), wobei die Oberfläche (12) eine Nadelschutzöffnung (14, 114) und einen freitragenden Arm (18) hat, der sich daraus erstreckt, wobei der Nadelschutz (10, 110) mit dem Injektorgehäuse (52, 152) nahe der Öffnung (54, 154a) der Oberfläche (54, 154) schwenkbar verbunden ist und zwischen einer ersten Stellung und einer zweiten Stellung beweglich ist; und
ein Vorspannelement (60), das mit der Injektionsnadel (58, 158) wirkmäßig verbunden ist, um den Nadelansatz (56, 156) und die Injektionsnadel (58, 158) von der eingezogenen Stellung zur Einspritzstellung axial zu verschieben,
**dadurch gekennzeichnet, dass**:
der Nadelansatz (56, 156) und die Injektionsnadel (58, 158) sich axial entlang einer Achse von der eingezogenen Stellung zur Einspritzstellung verschieben;
der freitragende Arm (18) ein geflanschtes freies Ende (18a) hat, das die Nadelschutzöffnung (14, 114) teilweise sperrt, die Injektionsnadel (58, 158) sich durch die Nadelschutzöffnung (14, 114) in der Einspritzstellung der Injektionsnadel (58, 158) und der ersten Stellung des Nadelschutzes (10, 110) erstreckt, das geflanschte freie Ende (18a) die Injektionsnadel (58, 158) in der ersten Stellung des Nadelschutzes (10, 110) freimacht, das geflanschte freie Ende (18a) die Injektionsnadel (58, 158) daran hindert, sich durch die Nadelschutzöffnung (14, 114) in der zweiten Stellung des Nadelschutzes (10, 110) zu erstrecken, und die Injektionsnadel (58, 158) den freitragenden Arm (18) elastisch ablenkt, um das geflanschte freie Ende (18a) beiseite zu bewegen und die Nadelschutzöffnung (14, 114) aufzuweiten, um dem Nadelschutz (10, 110) zu ermöglichen, über die Injektionsnadel (58, 158) hinaus zu schwenken und die zweite Stellung zu erreichen,
wenn der Nadelschutz (10, 110) in der zweiten Stellung ist und der Nadelansatz (56, 156) und die Injektionsnadel (58, 158) in der Einspritzstellung sind, das geflanschte freie Ende (18a) des Nadelschutzes (10, 110) die Spitze (58a, 158a) der Injektionsnadel (58, 158) deckt und die anschließende Bewegung des Nadelschutzes (10, 110) zur ersten Stellung den Nadelansatz (56, 156) und die Injektionsnadel (58, 158) zurück zur eingezogenen Stellung von der Einspritzstellung, entlang der Achse, axial verschiebt und
die Injektionsnadel (58, 158) eine Steifheit definiert, die größer ist als eine Steifheit des Vorspannelementes.

2. Injektor (50, 150) nach Anspruch 1, worin das Vorspannelement (60) eine Vorspannkraft aufwendet, um den Nadelansatz (56, 156) und die Injektionsnadel (58, 158) von der eingezogenen Stellung zur Einspritzstellung zu treiben, und wenn der Nadelschutz (10, 110) in der zweiten Stellung ist und der Nadelansatz (56, 156) und die Injektionsnadel (58, 158) in der Einspritzstellung sind, die anschließende Bewegung des Nadelschutzes (10, 110) von der zweiten Stellung zur ersten Stellung, mit einer Kraft, die der Vorspannkraft des Vorspannelementes entgegenwirkt und sie überwindet, den Nadelansatz (56, 156) und die Injektionsnadel (58, 158) zurück zur eingezogenen Stellung axial verschiebt.

3. Injektor (50, 150) nach Anspruch 1 oder Anspruch 2, worin, wenn der Nadelschutz (10, 110) in der zweiten Stellung ist und der Nadelansatz (56, 156) und die Injektionsnadel (58, 158) in der Einspritzstellung sind, der Nadelschutz (10, 110) die Injektionsnadel direkt berührt, um den Nadelansatz (56, 156) und die Injektionsnadel zurück zur eingezogenen Stellung, nach der anschließenden Bewegung des Nadelschutzes (10, 110) von der zweiten Stellung zur ersten Stellung, axial zu verschieben.

4. Injektor (50, 150) nach zumindest einem der vorhergehenden Ansprüche, worin, wenn der Nadelschutz (10, 110) in der zweiten Stellung ist und der Nadelansatz (56, 156) und die Injektionsnadel (58, 158) in der Einspritzstellung sind, das geflanschte freie Ende des freitragenden Armes die Spitze (58, 158) der Injektionsnadel (58, 158) berührt, um den Nadelansatz (56, 156) und die Injektionsnadel (58, 158) zurück zur eingezogenen Stellung, nach der anschließenden Bewegung des Nadelschutzes zur ersten Stellung, axial zu verschieben.

5. Injektor (50, 150) nach zumindest einem der vorhergehenden Ansprüche, worin der freitragende Arm elastisch flexibel ist, und worin der freitragende Arm flexibler als die Injektionsnadel (58, 158) ist.

6. Injektor (50, 150) nach zumindest einem der vorhergehenden Ansprüche, worin, wenn die Injektionsnadel (58, 158) in der Einspritzstellung ist, die Injektionsnadel (58, 158) mit dem freitragenden Arm in Eingriff kommt und ihn elastisch aus einer nicht gebeugten Ruhestellung davon während der Bewegung des Nadelschutzes (10, 110) von der ersten Stellung zur zweiten Stellung beugt, und worin die Injektionsnadel (58, 158) den freitragenden Arm in der zweiten Stellung des Nadelschutzes (10, 110) löst, wobei der freitragende Arm zur nicht gebeugten Ruhestellung davon zurückkehrt.

7. Injektor (50, 150) nach zumindest einem der vorhergehenden Ansprüche, worin, wenn die Injektionsnadel (58, 158) in der Einspritzstellung ist, die Injektionsnadel (58, 158) in das geflanschte freie Ende des freitragenden Armes während der Bewegung des Nadelschutzes (10, 110) von der ersten Stellung zur zweiten Stellung eingreift.

8. Injektor (50, 150) nach zumindest einem der vorhergehenden Ansprüche, worin, wenn der Nadelschutz (10, 110) in der zweiten Stellung ist und der Nadelansatz (56, 156) und die Injektionsnadel (58, 158) in der Einspritzstellung sind, die anschließende Bewegung des Nadelschutzes (10, 110) zur ersten Stellung indirekt die Injektionsnadel (58, 158) zurück zur eingezogenen Stellung axial verschiebt.

9. Injektor (50, 150) nach Anspruch 8, worin, wenn der Nadelschutz (10, 110) in der zweiten Stellung ist und der Nadelansatz (56, 156) und die Injektionsnadel (58, 158) in der Einspritzstellung sind, die anschließende Bewegung des Nadelschutzes (10, 110) zur ersten Stellung in den Arm des Nadelschutzes (10, 110) mit dem Nadelansatz (56, 156) eingreift, um den Nadelansatz (56, 156) und die Injektionsnadel (58, 158) zurück zur eingezogenen Stellung axial zu verschieben.

10. Injektor (50, 150) nach Anspruch 9, worin der Nadelansatz (56, 156) ein geflanschtes Element umfasst, und worin, wenn der Nadelschutz (10, 110) in der zweiten Stellung ist und der Nadelansatz (56, 156) und die Injektionsnadel (58, 158) in der Einspritzstellung sind, die anschließende Bewegung des Nadelschutzes (10, 110) zur ersten Stellung in den freitragenden Arm mit dem geflanschten Element des Nadelansatzes (56, 156) eingreift, um den Nadelansatz (56, 156) und die Injektionsnadel (58, 158) zurück zur eingezogenen Stellung axial zu verschieben.

11. Injektor (50, 150) nach Anspruch 9, worin das geflanschte freie Ende (18a) dafür ausgelegt ist, mit dem Nadelansatz (56, 156) in Eingriff zu kommen.

12. Injektor (50, 150) nach zumindest einem der vorhergehenden Ansprüche, worin der Nadelschutz (10, 110) im Allgemeinen mit der Oberfläche des Injektorgehäuses (52, 152) in der ersten Stellung bündig ist, und der Nadelschutz (10, 110) von der Oberfläche des Injektorgehäuses (52, 152) in der zweiten Stellung weggeschwenkt wird.

13. Injektor (50, 150) nach zumindest einem der vorhergehenden Ansprüche, worin der Nadelschutz (10, 110) von einer Kraft in die zweite Stellung davon vorgespannt wird.

14. Injektor (50, 150) nach zumindest einem der vorhergehenden Ansprüche, worin das Vorspannelement (60) eine Schraubenfeder ist, die von einem zusammengefahrenen Zustand zu einem ausgefahrenen Zustand ausfahrbar ist, um den Nadelansatz (56, 156) und die Injektionsnadel (58, 158) von der eingezogenen Stellung zur Einspritzstellung axial zu verschieben.

15. Injektor (50, 150) nach Anspruch 14, worin, wenn der Nadelschutz (10, 110) in der zweiten Stellung ist und der Nadelansatz (56, 156) und die Injektionsnadel (58, 158) in der Einspritzstellung sind, die anschließende Bewegung des Nadelschutzes (10, 110) zur ersten Stellung, die den Nadelansatz (56, 156) und die Injektionsnadel (58, 158) zurück zur eingezogenen Stellung axial verschiebt, auch die Schraubenfeder zurück zum zusammengefahrenen Zustand zusammendrückt.

## Revendications

1. Injecteur (50, 150) comprenant :
un boîtier d'injecteur (52, 152) présentant une surface (54, 154) configurée pour venir en contact avec une surface cutanée d'un utilisateur, la surface (54, 154) présentant une ouverture (54a, 154a) ;
une embase d'aiguille (56, 156) montée mobile à l'intérieur du boîtier d'injecteur (52, 152) ;
une aiguille d'injection (58, 158) portée par l'embase d'aiguille mobile (56, 156), l'embase d'aiguille (56, 156) et l'aiguille d'injection (58, 158) pouvant se déplacer en translation axiale entre une position rétractée, dans laquelle au moins une pointe (58a, 158a) de l'aiguille d'injection (58, 158) est contenue à l'intérieur du boîtier d'injecteur (52, 152), et une position d'injection, dans laquelle au moins la pointe (58a, 158a) de l'aiguille d'injection (58, 158) fait saillie hors du boîtier d'injecteur (52, 152) ;
un protège-aiguille (10, 110) comprenant une surface (12), la surface (12) présentant un orifice de protège-aiguille (14, 114) et un bras en porte-à-faux (18) s'étendant à partir de celle-ci, le protège-aiguille (10, 110) étant relié de manière pivotante au boîtier d'injecteur (52, 152) à proximité de l'ouverture (54, 154a) de la surface (54, 154) et mobile entre une première position et une seconde position ; et
un organe de sollicitation (60) relié fonctionnellement à l'aiguille d'injection (58, 158) pour déplacer en translation axiale l'embase d'aiguille (56, 156) et l'aiguille d'injection (58, 158) de la position rétractée à la position d'injection,
**caractérisé en ce que** :
l'embase d'aiguille (56, 156) et l'aiguille d'injection (58, 158) se déplacent en translation axiale le long d'un axe, de la position rétractée à la position d'injection ;
le bras en porte-à-faux (18) présente une extrémité libre à rebord (18a) obstruant partiellement l'orifice de protège-aiguille (14, 114), l'aiguille d'injection (58, 158) s'étend à travers l'orifice de protège-aiguille (14, 114) dans la position d'injection de l'aiguille d'injection (58, 158) et dans la première position du protège-aiguille (10, 110), l'extrémité libre à rebord (18a) laissant le passage libre à l'aiguille d'injection (58, 158) dans la première position du protège-aiguille (10, 110), l'extrémité libre à rebord (18a) empêchant l'aiguille d'injection (58, 158) de s'étendre à travers l'orifice de protège-aiguille (14, 114) dans la seconde position du protège-aiguille (10, 110), et l'aiguille d'injection (58, 158) faisant fléchir élastiquement le bras en porte-à-faux (18) pour écarter l'extrémité libre à rebord (18a) et agrandir l'orifice de protège-aiguille (14, 114) afin de permettre au protège-aiguille (10, 110) de pivoter au-delà de l'aiguille d'injection (58, 158) et d'atteindre la seconde position,
lorsque le protège-aiguille (10, 110) est dans la seconde position et que l'embase d'aiguille (56, 156) et l'aiguille d'injection (58, 158) sont dans la position d'injection, l'extrémité libre à rebord (18a) du protège-aiguille (10, 110) recouvre la pointe (58a, 158a) de l'aiguille d'injection (58, 158) et un déplacement ultérieur du protège-aiguille (10, 110) vers la première position ramène l'embase d'aiguille (56, 156) et l'aiguille d'injection (58, 158) en translation axiale de la position d'injection vers la position rétractée, le long de l'axe, et
l'aiguille d'injection (58, 158) définit une raideur supérieure à une raideur de l'organe de sollicitation.

2. Injecteur (50, 150) selon la revendication 1, dans lequel l'organe de sollicitation (60) applique une force de sollicitation pour entraîner l'embase d'aiguille (56, 156) et l'aiguille d'injection (58, 158) de la position rétractée à la position d'injection, et, lorsque le protège-aiguille (10, 110) est dans la seconde position et que l'embase d'aiguille (56, 156) et l'aiguille d'injection (58, 158) sont dans la position d'injection, un déplacement ultérieur du protège-aiguille (10, 110) de la seconde position vers la première position, avec une force qui s'oppose à la force de sollicitation de l'organe de sollicitation et qui la surmonte, ramène l'embase d'aiguille (56, 156) et l'aiguille d'injection (58, 158) en translation axiale vers la position rétractée.

3. Injecteur (50, 150) selon la revendication 1 ou la revendication 2, dans lequel, lorsque le protège-aiguille (10, 110) est dans la seconde position et que l'embase d'aiguille (56, 156) et l'aiguille d'injection (58, 158) sont dans la position d'injection, le protège-aiguille (10, 110) vient directement en contact avec l'aiguille d'injection pour ramener l'embase d'aiguille (56, 156) et l'aiguille d'injection en translation axiale vers la position rétractée, lors d'un déplacement ultérieur du protège-aiguille (10, 110) de la seconde position vers la première position.

4. Injecteur (50, 150) selon au moins l'une des revendications précédentes, dans lequel, lorsque le protège-aiguille (10, 110) est dans la seconde position et que l'embase d'aiguille (56, 156) et l'aiguille d'injection (58, 158) sont dans la position d'injection, l'extrémité libre à rebord du bras en porte-à-faux vient en contact avec la pointe (58, 158) de l'aiguille d'injection (58, 158) pour ramener l'embase d'aiguille (56, 156) et l'aiguille d'injection (58, 158) en translation axiale vers la position rétractée, lors d'un déplacement ultérieur du protège-aiguille vers la première position.

5. Injecteur (50, 150) selon au moins l'une des revendications précédentes, dans lequel le bras en porte-à-faux est élastiquement flexible, et dans lequel le bras en porte-à-faux est plus flexible que l'aiguille d'injection (58, 158).

6. Injecteur (50, 150) selon au moins l'une des revendications précédentes, dans lequel, lorsque l'aiguille d'injection (58, 158) est dans la position d'injection, l'aiguille d'injection (58, 158) vient en prise avec le bras en porte-à-faux et le fait fléchir élastiquement hors d'une position de repos non fléchie de ce dernier pendant un déplacement du protège-aiguille (10, 110) de la première position à la seconde position, et dans lequel l'aiguille d'injection (58, 158) cesse d'être en prise avec le bras en porte-à-faux dans la seconde position du protège-aiguille (10, 110), moyennant quoi le bras en porte-à-faux revient à sa position de repos non fléchie.

7. Injecteur (50, 150) selon au moins l'une des revendications précédentes, dans lequel, lorsque l'aiguille d'injection (58, 158) est dans la position d'injection, l'aiguille d'injection (58, 158) vient en prise avec l'extrémité libre à rebord du bras en porte-à-faux pendant un déplacement du protège-aiguille (10, 110) de la première position à la seconde position.

8. Injecteur (50, 150) selon au moins l'une des revendications précédentes, dans lequel, lorsque le protège-aiguille (10, 110) est dans la seconde position et que l'embase d'aiguille (56, 156) et l'aiguille d'injection (58, 158) sont dans la position d'injection, un déplacement ultérieur du protège-aiguille (10, 110) vers la première position ramène indirectement l'aiguille d'injection (58, 158) en translation axiale vers la position rétractée.

9. Injecteur (50, 150) selon la revendication 8, dans lequel, lorsque le protège-aiguille (10, 110) est dans la seconde position et que l'embase d'aiguille (56, 156) et l'aiguille d'injection (58, 158) sont dans la position d'injection, un déplacement ultérieur du protège-aiguille (10, 110) vers la première position met le bras du protège-aiguille (10, 110) en prise avec l'embase d'aiguille (56, 156) pour ramener l'embase d'aiguille (56, 156) et l'aiguille d'injection (58, 158) en translation axiale vers la position rétractée.

10. Injecteur (50, 150) selon la revendication 9, dans lequel l'embase d'aiguille (56, 156) comprend un élément à rebord, et dans lequel, lorsque le protège-aiguille (10, 110) est dans la seconde position et que l'embase d'aiguille (56, 156) et l'aiguille d'injection (58, 158) sont dans la position d'injection, un déplacement ultérieur du protège-aiguille (10, 110) vers la première position met le bras en porte-à-faux en prise avec l'élément à rebord de l'embase d'aiguille (56, 156) pour ramener l'embase d'aiguille (56, 156) et l'aiguille d'injection (58, 158) en translation axiale vers la position rétractée.

11. Injecteur (50, 150) selon la revendication 9, dans lequel l'extrémité libre à rebord (18a) est configurée pour venir en prise avec l'embase d'aiguille (56, 156).

12. Injecteur (50, 150) selon au moins l'une des revendications précédentes, dans lequel le protège-aiguille (10, 110) affleure globalement la surface du boîtier d'injecteur (52, 152) dans la première position, et le protège-aiguille (10, 110) est écarté, par pivotement, de la surface du boîtier d'injecteur (52, 152) dans la seconde position.

13. Injecteur (50, 150) selon au moins l'une des revendications précédentes, dans lequel le protège-aiguille (10, 110) est rappelé par une force dans sa seconde position.

14. Injecteur (50, 150) selon au moins l'une des revendications précédentes, dans lequel l'organe de sollicitation (60) est un ressort hélicoïdal extensible d'un état contracté à un état étendu pour déplacer en translation axiale l'embase d'aiguille (56, 156) et l'aiguille d'injection (58, 158) de la position rétractée à la position d'injection.

15. Injecteur (50, 150) selon la revendication 14, dans lequel, lorsque le protège-aiguille (10, 110) est dans la seconde position et que l'embase d'aiguille (56, 156) et l'aiguille d'injection (58, 158) sont dans la position d'injection, un déplacement ultérieur du protège-aiguille (10, 110) vers la première position, déplacement qui ramène l'embase d'aiguille (56, 156) et l'aiguille d'injection (58, 158) en translation axiale vers la position rétractée, comprime également le ressort hélicoïdal de nouveau vers l'état contracté.
